# EUROPÄISCHE PATENTANMELDUNG

(11) **EP 1 312 368 A1**
(43) Veröffentlichungstag der Anmeldung: **21.05.2003**
(21) Anmeldenummer: 01127306.7
(22) Anmeldetag: 19.11.2001
(51) Int. Cl.: A61K 31/606, A61K 9/12

(54) **Zusammensetzungen enthaltend Mesalazin zur Behandlung von entzündlichen Darmerkrankungen**

(71) Anmelder: DR. FALK PHARMA GMBH, D-79108 Freiburg (DE)
(72) Erfinder: Kühn, Reimund, Dr., 79348 Freiamt (DE)
(74) Vertreter: Keller, Günter, Dr.

(57) **Zusammenfassung**

Die vorliegende Erfindung betrifft eine Zusammensetzung zur rektalen Behandlung entzündlicher Darmerkrankungen enthaltend 5 bis 50% Mesalazin und 50-95% eines mit Wasser mischbaren flüssigen Vehikels, das im wesentlichen Propylenglykol und/oder Glycerin ist, wobei die Zusammensetzung im wesentlichen wasserfrei ist und bei Verabreichung einen Schaum bildet. Die Erfindung umfaßt auch die Verwendung von Propylenglykol und/oder Glycerin zur Herstellung derartiger Arzneimittel.

## Beschreibung

Die vorliegende Erfindung betrifft das Gebiet der Behandlung entzündlicher Darmerkrankungen. Entzündliche Erkrankungen des Darms sind insbesondere Colitis ulcerosa und Morbus Crohn. Diese Krankheiten sind chronisch und treten häufig erstmals in den ersten Lebensjahrzehnten auf.

Colitis ulcerosa ist eine Erkrankung des Kolons, die zur Entzündung und Bildung von Geschwüren der Mukosa des Dickdarms und des Rektums führt. Die Krankheit ist relativ verbreitet in der westlichen Welt. Sie tritt am häufigsten bei Menschen im Alter zwischen 15 und 30 Jahren auf, obwohl auch Kinder und Ältere gelegentlich erkranken. Über 50% der Patienten sind zu bestimmten Zeiten frei von Symptomen, die sehr große Mehrheit allerdings erleidet wenigstens einen Krankheits-"Schub" innerhalb eines Zeitraums von 10 Jahren.

Bei diesen Entzündungserkrankungen kommt es darauf an, einen Wirkstoff zur Behandlung möglichst nahe auf die von der Entzündung betroffenen Schleimhäute zu bringen. Dort soll der Wirkstoff von der entzündeten Darmregion aufgenommen und damit wirksam werden. Eine Resorption und damit systemische Verfügbarkeit des Wirkstoffs soll so weit wie möglich vermieden werden, um die systemische Belastung und mögliche Nebenwirkungen zu vermeiden.

Die Verwendung von Mesalazin (5-Aminosalicylsäure) zur Behandlung entzündlicher Darmerkrankungen, insbesondere der Colitis ulcerosa, ist seit langem bekannt (Azad-Khan et al., Lancet, 29th Oct. 1977, 892-895). Neben der oralen Behandlung mit spezifisch freisetzenden Produkten werden auch rektal zu applizierende Präparate eingesetzt, vor allem zur Behandlung von Erkrankungen der distalen Darmabschnitte. Bekannt ist die rektale Anwendung entzündungshemmender Substanzen in Form von Suppositorien, Klystieren und Enemas.

Suppositorien haben den Nachteil, daß ein lokal wirksamer Wirkstoff nur die Enddarmregion, nämlich das Rektum, erreicht, eine Ausspreitung in höhere Darmabschnitte ist nicht gegeben.

Um diesen Nachteil zu beseitigen, wurden Klystiere und Enemas entwickelt. Auf Grund der im Vergleich zu Suppositorien größeren Volumina kann sich der Wirkstoff naturgemäß weiter ausbreiten und auch von der Entzündung betroffene Schleimhautpartien in höher gelegenen Darmabschnitten, wie dem Kolon und dem Sigma erreichen. Der Nachteil dieser Zubereitungen ist der durch größere Volumina - verwendet werden Volumina von 50-250 ml - ausgeübte Reiz auf den Dann, der besonders bei erkrankten Personen zu Stuhldrang führt. Dadurch wird das Medikament vorzeitig ausgeschieden. Auf Grund der kurzen Verweildauer kann keine ausreichende Wirksamkeit gewährleistet werden.

Zur Vermeidung der beschriebenen Nachteile wurden Mesalazin-Schäume entwickelt. EP 0 395 329 A2 beschreibt einen Mesalazin-haltigen Schaum auf wässriger Basis. Es werden Volumina von 2-5 ml verabreicht. Aufgrund des geringen Volumens dürften ausschließlich die untersten Darmabschnitte erreicht werden.

EP 0 468 555 A1 beschreibt ebenfalls Schäume, die Mesalazin enthalten können. Das Problem mangelhaften Aufsteigens in höhere Darmabschnitte wird gelöst, indem Mengen größer als 100 ml appliziert werden, mit dem Nachteil, daß der von den Enemas und Klystieren bekannte Nachteil des ungenügend langen Haltens des Präparates auftritt und der Wirkstoff ausgeschieden wird, bevor er vollständig wirksam werden kann. Ein solches Präparat ist unter dem Namen Asacol®-Schaum in Italien im Handel.

Weitere Nachteile von Schäumen auf wässriger Basis sind chemisch-technischer Art:
- Um ein Keimwachstum im wässrigen Milieu zu vermeiden, ist die Verwendung von Konservierungsmitteln unumgänglich. Diese können insbesondere bei entzündeten Schleimhäuten zu unangenehmen Irritationen und zu allergischen Reaktionen führen.
- Mesalazin ist relativ instabil, insbesondere in Lösung. In Wasser ist Mesalazin teilweise löslich. Zusammen mit im Vehikel enthaltenem Sauerstoff tritt Oxidation auf. Dies führt zu starken Verfärbungen und macht das Produkt schon nach kurzer Lagerzeit unbrauchbar. Der Zusatz von Antioxidantien wie z.B. Sulfit ist unumgänglich. Wegen möglicher Sulfit-Allergie sollten Antioxidatien jedoch in pharmazeutischen Produkten möglichst vermieden werden.
- Aufgrund der teilweisen Löslichkeit des Mesalazins tritt in Wasser Partikelwachstum auf. Im wasserfreien Schaum sind nur etwa 14 ppm gelöst, im vergleichbaren wässrigen Vehikel etwa 14.000 ppm. Es können lange Wirkstoffnadeln entstehen, die die Wirksamkeit des ursprünglichen in kleiner Teilchengröße vorliegenden Wirkstoffs reduzieren und außerdem zur Verstopfung der Ventilauslaßöffnung des Applikators führen können.
- Da aus Stabilitätsgründen - Mesalazin ist sehr alkaliempfindlich - wässrige Vehikel sauer eingestellt werden müssen, können bei längerer Lagerung die aus Aluminium bestehenden Applikationsbehältnisse leicht korrodieren.

Eine Aufgabe der vorliegenden Erfindung ist es, ein vorteilhaftes Arzneimittel zur Behandlung von entzündlichen Darmerkrankungen bereitzustellen.

Überraschenderweise wurde gefunden, daß Schäume mit einem im wesentlichen wasserfreien Vehikel, beruhend auf einer mit Wasser mischbaren Flüssigkeit, eine gute Spreitfähigkeit zeigen und einen oder mehrere der genannten chemisch-technischen Nachteile nicht aufweisen. Die Erfindung betrifft daher eine Zusammensetzung zur intrarektalen Behandlung entzündlicher Darmerkrankungen enthaltend 5 bis 50% Mesalazin und 50-95 % eines mit Wasser mischbaren flüssigen Vehikels, wobei die Zusammensetzung im wesentlichen wasserfrei ist und bei Verabreichung einen Schaum bildet.

Das mit Wasser mischbare flüssige Vehikel ist im wesentlichen Propylenglykol und/oder Glycerin. Das Vehikel kann aus einem der zwei genannten Alkohole bestehen oder aus einem Gemisch der zwei genannten Alkohole. Bevorzugt ist es, daß das Vehikel im wesentlichen aus einem der zwei genannten Alkohole besteht, am bevorzugtesten ist dabei Propylenglykol.

Wesentlich für die vorliegende Erfindung ist, daß die Zusammensetzung im wesentlichen wasserfrei ist. Das bedeutet im Rahmen der Erfindung, daß die Zusammensetzung weniger als 5 % Wasser, bevorzugt weniger als 2,5 % Wasser, bevorzugter weniger als 1 % Wasser, am bevorzugtesten kein Wasser (d.h. weniger als 0,1 % Wasser) enthält. Dem Fachmann ist aber klar, daß kommerziell erhältliche Chemikalien oft Spuren von Wasser enthalten. Sehr geringe Wassermengen, die so über andere Chemikalien in die Zusammensetzung gelangen können, stehen dem Merkmal "im wesentlichen wasserfrei" nicht entgegen.

Die Konzentration des Wirkstoffs Mesalazin in der Zusammensetzung ist 5 bis 50 %, vorzugsweise 10 bis 40 %, bevorzugter 15 bis 30 %. Am bevorzugtesten ist die Mesalazin-Konzentration 20 bis 25 %. Alle Prozentangaben in der vorliegenden Anmeldung, soweit nicht anders angegeben, sind Gewichts-%, d.h. % bezogen auf den zu applizierenden Schaum.

Die Konzentration des mit Wasser mischbaren Vehikels, insbesondere von Propylenglykol, in der Zusammensetzung ist 50 bis 95 %, bevorzugt 60 bis 90 %, bevorzugter 70 bis 85 %, am bevorzugtesten 75 bis 80 %.

Die Konzentration des Wirkstoffs Mesalazin und des Vehikels zusammen in der erfindungsgemäßen Zusammensetzung ist üblicherweise größer als 90 %, bevorzugt größer als 95 %, am bevorzugtesten größer als 97 %. Das bedeutet, daß nur sehr geringe Mengen weiterer Zusatzstoffe bzw. Schaumbildner notwendig sind, um vorteilhafte Zusammensetzungen zu erhalten. Dies ist gegenüber dem Stand der Technik von Vorteil, da bisher nur Schäume beschrieben sind, die Anteile von Schaumbildnern, Dickungsmittel und Konservierungsmitteln in einem Gesamtanteil von ca. 5-45 % verwenden.

Die Spreitfähigkeit der Zusammensetzung kann weiter erhöht werden durch den Zusatz von Emulgatoren oder durch den Zusatz von sogenannten Spreitmitteln, wie z. B.

Lecithinen bzw. Hostaphaten. Letztere sind synthetische Tenside, die den natürlichen Lecithinen nachempfunden sind. Beispiele für Lecithine bzw. Hostaphate sind Lecithin ex ovo bzw. Phosphatidylcholin. Bekannte Emulgatoren sind Polysorbat und Cetylstearylalkohol.

Die erfindungsgemäße Zusammensetzung kann in einem geeigneten Behältnis bereitgestellt werden, aus dem dosisweise bestimmte Mengen an Schaum entnommen werden können. Dies wird vorzugsweise durch eine an dem Behältnis angebrachte Dosiereinrichtung, üblicherweise ein Dosierventil, erreicht. Das Behältnis kann auch ein besonderes Mittel aufweisen, das die rektale Applikation der Zusammensetzung ermöglicht. Das Behältnis mit der Dosiereinrichtung ist so ausgestaltet, daß bei Betätigung der Dosiereinrichtung zur Entnahme einer Dosis 5 bis 70 ml Schaum zur Verfügung gestellt werden, bevorzugt 10 bis 60 ml Schaum, bevorzugter 15 bis 40 ml Schaum, am bevorzugtesten 20 bis 30 ml Schaum. Bei bestimmungsgemäßem Gebrauch erfolgt die Aufschäumung im Darm. Die Menge an Flüssigkeit (3 - 6 ml), die über die Dosiereinrichtung appliziert wird, bildet demzufolge am bevorzugtesten 20 - 30 ml Schaum.

Das Behältnis hat üblicherweise ein Fassungsvolumen von 50 bis 150 ml, vorzugsweise von 75 bis 125 ml, am bevorzugtesten von ungefähr 100 ml.

Wenn die Zusammensetzung in einem Behältnis, beispielsweise in einem Metallbehältnis, vorzugsweise aus Aluminium, bereitgestellt wird, enthält die Zusammensetzung ein Treibmittel. Bekannte Treibmittel sind Kohlenwasserstoffe wie z. B. Isobutan, n-Butan und/oder Propan oder Stickstoff. Die Zusammensetzung steht dabei in dem Behältnis unter Druck, z. B. unter einem Druck von ungefähr 2,5 bar. Die Menge des Treibmittels in der Zusammensetzung ist 1 bis 10 %, vorzugsweise 2 bis 8 %, am bevorzugtesten 3 bis 6 %, bezogen auf die Gesamtmenge der Zusammensetzung einschließlich des Treibmittels. Alle übrigen %-Angaben in der vorliegenden Anmeldung, insbesondere die des Wirkstoffs Mesalazin und des Vehikels, beziehen sich auf die Gesamtmenge der Zusammensetzung ohne Treibmittel.

Ein weiterer Aspekt der Erfindung ist ein Schaum, den man bei Anwendung der erfindungsgemäßen Zusammensetzung erhält.

Die erfindungsgemäßen Zusammensetzungen und Schäume sind geeignet zur Behandlung von chronischen entzündlichen Darmerkrankungen und entzündlichen Zuständen des Darmes, und besonders geeignet zur Behandlung von Colitis ulcerosa. Die Behandlung kann in allen Entzündungs- und Aktivitätsstadien der Colitis ulcerosa erfolgen. Die Erfindung umfaßt auch die Verwendung der erfindungsgemäßen Zusammensetzungen und Schäume zur Erhaltung der Remission in den inaktiven Phasen der Erkrankung.

Die Erfindung betrifft außerdem die Verwendung von Propylenglykol und/oder Glycerin zur Herstellung eines Arzneimittels zur Behandlung von entzündlichen Darmerkrankungen, dadurch gekennzeichnet, daß das Arzneimittel 5 bis 50% Mesalazin und 50-95% eines mit Wasser mischbaren flüssigen Vehikels, das im wesentlichen Propylenglykol und/oder Glycerin ist, enthält, im wesentlichen wasserfrei ist und bei Verabreichung einen Schaum bildet. Die bevorzugten Ausführungsformen der Verwendung entsprechen den bevorzugten Ausführungsformen der Zusammensetzung, wie sie in der vorliegenden Anmeldung beschrieben sind.

Die erfindungsgemäßen Zusammensetzungen weisen den überraschenden Vorteil auf, daß aufgrund ihrer guten Spreitfähigkeit höhere Darmabschnitte mit einem geringeren Volumen an Schaum bzw. Schaumbildner erreicht werden können als dies bei Schäumen auf wässriger Basis der Fall ist. Schon Schaumvolumina ≤ 60 ml Schaum entsprechend etwa 6 ml Schaumbildner erreichen aufgrund der guten Spreitfähigkeit im Vergleich zu wässrigen Vehikeln Darmabschnitte bis zum proximalen Sigma und Colon descendens.

Für den erfindungsgemäßen Mesalazin-Schaum konnte in einer Szintigraphiestudie gezeigt werden, daß eine Ausbreitung des Schaumes bei Patienten über das Colon descendens bis zum Colon ascendens erreicht wurde. Die Persistenz des Schaumes in der jeweiligen Dickdarmregion war in dieser Studie besser als die Persistenz des Enemas. Persistenz ist die Dauerhaftigkeit der Arzneimitteleinwirkung am betreffenden Darmabschnitt (siehe Beispiel 3).

Die erfindungsgemäßen Zusammensetzungen führen zu einem feinporigen Schaum, der auf der Schleimhautoberfläche zerfällt und den Wirkstoff feinstverteilt auf diese freisetzt.

Das niedrige Schaumvolumen pro Dosis, bevorzugt 20 - 30 ml, erzeugt keinen Stuhldrang und damit vorzeitiges Ausscheiden des Wirkstoffs.

Aufgrund des niedrigen Volumens pro Dosis lassen sich problemlos mehrere Applikationen (10 - 20) in einem handlichen Behältnis unterbringen. Das führt u.a. zu niedrigeren Herstellungskosten im Vergleich zu den sonst notwendigen 10 - 20 Eindosen-Applikationssystemen. Die bakteriostatische Wirkung des Vehikels Propylenglykol ermöglicht es, nicht nur auf den Zusatz von Konservierungsmitteln zu verzichten, sondern hat auch einen zusätzlichen therapeutischen Effekt auf die entzündete Darmschleimhaut, die häufig bakterielle Infektionen zeigt.

Stabilitätsprobleme, die bei wässrigen Systemen auftreten, wie
- oxidative Zersetzung und damit Verfärbungen des Wirkstoffs,
- mit der Teillöslichkeit verbundenes Kristallwachstum und
- Korrosion des Behältermaterials
können vermieden werden.

Die nachfolgenden Beispiele erläutern die Erfindung näher.

### Beispiel 1

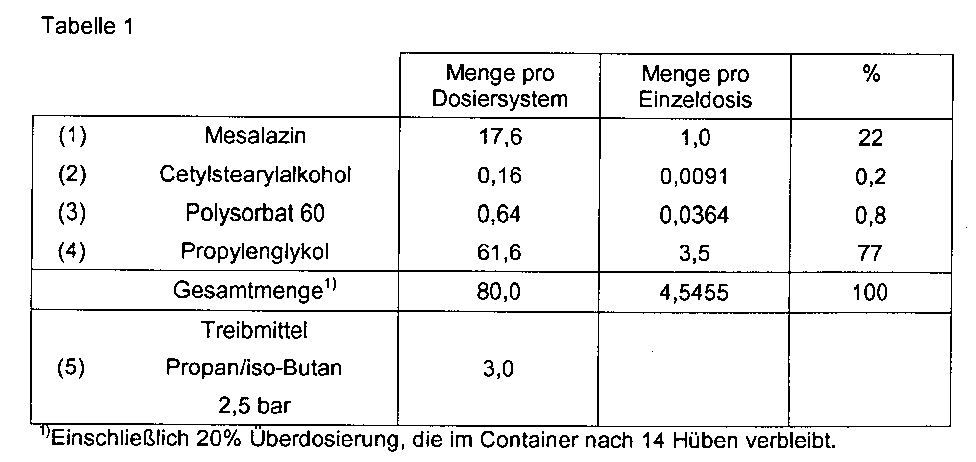

(2) uns (3) werden unter gelindem Erwärmen in (4) gelöst. Nach dem Abkühlen wird der Wirkstoff (1) in der Dispersion suspendiert. Die Menge pro Dosiersystem, das sind 80 g (entspricht 14 Hübe + 20 % Überdosierung) werden in Aluminium-Behälter mit einem Volumen von ca. 100 ml abgefüllt. Das Gefäß wird mit einem entsprechenden Dosierventil (z.B. Lablabo 1" valve aus Polyester) verschlossen. Das Treibmittel Propan/iso-Butan 3g wird zugegeben.

### Beispiel 2:

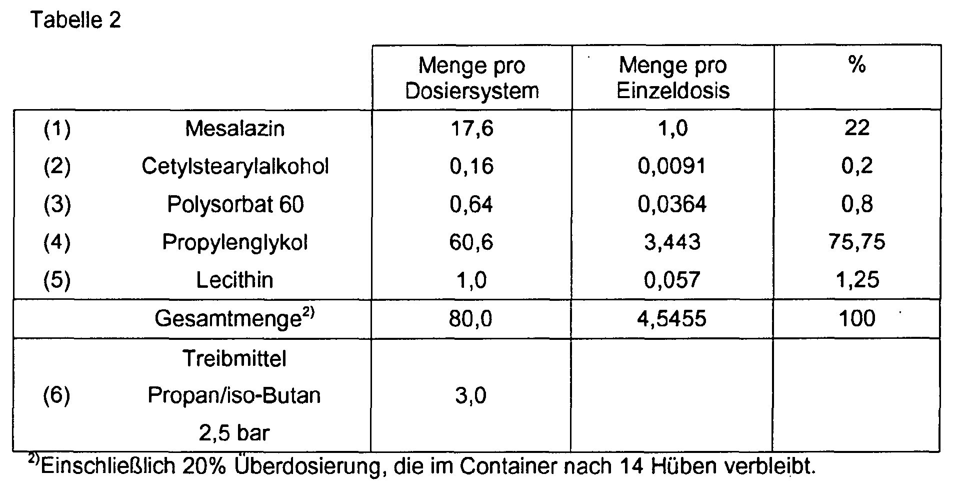

(2), (3) und (5) werden unter gelindem Erwärmen in (4) gelöst. Nach dem Abkühlen wird der Wirkstoff (1) in der Dispersion suspendiert. Die Menge pro Dosiersystem, das sind 80 g (entspricht 14 Hübe + 20 % Überdosierung) werden in Aluminium-Behälter mit einem Volumen von ca. 100 ml abgefüllt. Das Gefäß wird mit einem entsprechenden Dosierventil (z.B. Lablabo 1" valve aus Polyester) verschlossen. Das Treibmittel Propan/iso-Butan 3g wird zugegeben.

### Beispiel 3:

Persistenzvergleich zwischen dem erfindungsgemäßen Schaum und einem Vergleichsenema bei Patienten:

Es wurde eine szintigraphische Studie über die Verteilung von Samarium (¹⁵³Sm)-markiertem Mesalazinschaum und Mesalazinenema im Kolon von sechs Patienten mit Colitis ulcerosa durchgeführt. Der Schaum und das Enema enthielten die gleiche Konzentration (2 g/60 ml) an markiertem Mesalazin.

Nach Verabreichung des Schaums und des Enemas wurden szintigraphische Aufnahmen am Abdomen von sechs Patienten gemacht. Dazu wurden Scans mit einer Gamma-Kamera durchgeführt. Es wurden Scans 5 min nach Verabreichung, sowie 0,5, 1, 2, 4, 6 und 12 h nach Verabreichung aufgenommen. Das Abdomen wurde in 5 Bereiche eingeteilt: Colon ascendens, Colon transversum, Colon descendens, Colon sigmoideum und Rektum.

Anschließend wurden die Bilder auf einen Datenträger übertragen, mittels einer geeigneten Software wurden 5 "Regions of Interest" (ROIs) generiert, die den 5 oben genannten anatomischen Regionen des Abdomens entsprachen. Die rechteckigen ROIs wurden gespeichert und auf jedes der gewonnenen Szintigramme angewendet. Die gemessenen Radioaktivitätswerte für jede Region wurden in jedem Zeitintervall aufgezeichnet.

Die **Persistenz** des Wirkstoffs in den Darmbereichen wurde dadurch ermittelt, daß die Radioaktivitätswerte der Scans nach 4, 6 und 12 h mit dem besten Scan innerhalb der ersten 2 Stunden verglichen wurden. Zur Bewertung der Persistenz wurde eine Bewertungsskala von 0 bis 3 benutzt (0=keine Persistenz, 1=geringe Persistenz, 2=mittlere Persistenz, 3=gute Persistenz).

Für jeden der 6 Patienten wurden somit Persistenzwerte für die 5 verschiedenen Bereiche erhalten. Die Einzelwerte der 6 Patienten wurden addiert. Folgende Ergebnisse wurden erhalten:

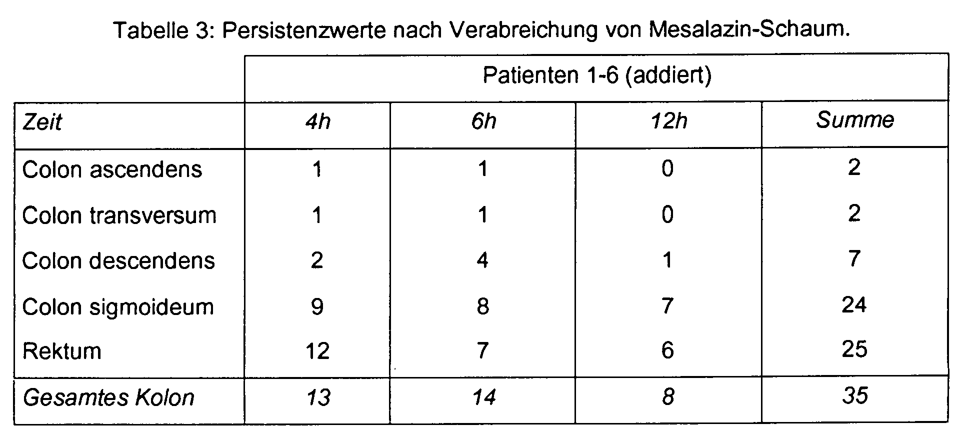

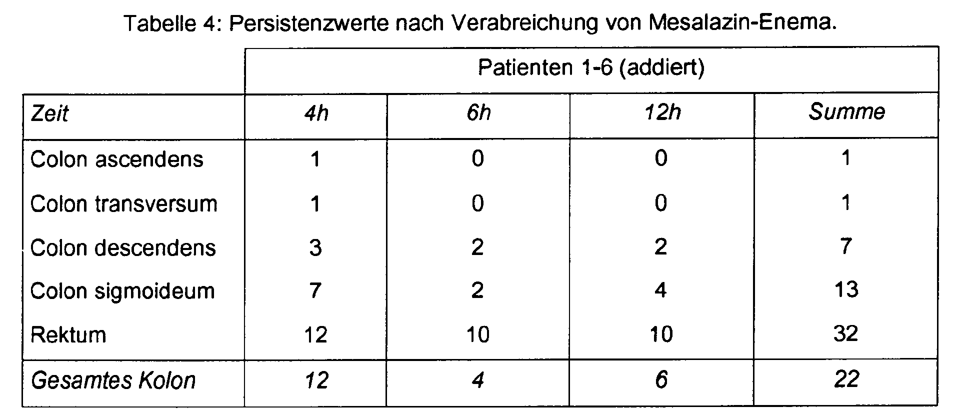

Die Ergebnisse zeigen, daß der erfindungsgemäße Mesalazinschaum gegenüber dem Vergleichsenema eine überlegene Persistenz im Kolon aufweist.

## Patentansprüche

1. Zusammensetzung zur intrarektalen Behandlung entzündlicher Darmerkrankungen enthaltend
- 5 bis 50% Mesalazin und
- 50-95% eines mit Wasser mischbaren flüssigen Vehikels, das im wesentlichen Propylenglykol und/oder Glycerin ist,
**dadurch gekennzeichnet, daß** die Zusammensetzung im wesentlichen wasserfrei ist und bei Verabreichung einen Schaum bildet.

2. Zusammensetzung nach Anspruch 1, **dadurch gekennzeichnet, daß** das mit Wasser mischbare flüssige Vehikel im wesentlichen Propylenglykol ist.

3. Zusammensetzung nach Anspruch 1 oder 2, **dadurch gekennzeichnet, daß** sie 20-25% Mesalazin enthält.

4. Zusammensetzung nach einem der Ansprüche 1 bis 3, **dadurch gekennzeichnet, daß** sie 75-80% des mit Wasser mischbaren flüssigen Vehikels enthält.

5. Zusammensetzung nach einem der Ansprüche 1 bis 4, **dadurch gekennzeichnet, daß** die Konzentration von Mesalazin und Vehikel zusammen in der Zusammensetzung größer als 97% ist.

6. Zusammensetzung nach einem der Ansprüche 1 bis 5, **dadurch gekennzeichnet, daß** sie weiterhin einen oder mehrere Bestandteile enthält, die ausgewählt sind aus Emulgatoren, Spreitmitteln und Surfactants.

7. Zusammensetzung nach einem der Ansprüche 1 bis 6, **dadurch gekennzeichnet, daß** die entzündliche Darmerkrankung Colitits ulcerosa ist.

8. Zusammensetzung nach einem der Ansprüche 1 bis 7, **dadurch gekennzeichnet, daß** sie in einem Behältnis bereitgestellt wird, das ein Dosierventil aufweist.

9. Zusammensetzung nach Anspruch 8, **dadurch gekennzeichnet, daß** bei Betätigung des Dosierventils eine Dosis zur Verfügung gestellt wird, die weniger als 60 ml Schaum entspricht.

10. Zusammensetzung nach einem der vorhergehenden Ansprüche, **dadurch gekennzeichnet, daß** sie ein Treibmittel enthält.

11. Schaum zur Behandlung entzündlicher Darmerkrankungen erhältlich aus einer Zusammensetzung nach einem der Ansprüche 1 bis 10.

12. Verwendung von Propylenglykol und/oder Glycerin zur Herstellung eines Arzneimittels zur Behandlung von entzündlichen Darmerkrankungen, **dadurch gekennzeichnet, daß** das Arzneimittel 5 bis 50% Mesalazin und 50-95% eines mit Wasser mischbaren flüssigen Vehikels, das im wesentlichen Propylenglykol und/oder Glycerin ist, enthält, im wesentlichen wasserfrei ist und bei Verabreichung einen Schaum bildet.
